# EUROPÄISCHE PATENTSCHRIFT

(11) **EP 4 340 918 B1**
(45) Veröffentlichungstag und Bekanntmachung des Hinweises auf die Patenterteilung: **13.08.2025**
(21) Anmeldenummer: 22728674.7
(22) Anmeldetag: 16.05.2022
(51) Int. Cl.: A61M 13/00, A61M 16/08, A61M 39/10, A61M 39/14, F16L 37/56

(54) **SCHLAUCHVERBINDUNGSVORRICHTUNG FÜR MEDIZINTECHNISCHE PUMPEN, INSBESONDERE FÜR INSUFFLATIONSPUMPEN FÜR DIE ENDOSKOPIE**
HOSE CONNECTING DEVICE FOR MEDICAL PUMPS, IN PARTICULAR FOR INSUFFLATION PUMPS FOR ENDOSCOPY
DISPOSITIF DE RACCORDEMENT DE TUYAUX POUR POMPES MÉDICALES, EN PARTICULIER POUR POMPES D'INSUFFLATION POUR L'ENDOSCOPIE

(30) Priorität: 17.05.2021 DE 102021002721
(43) Veröffentlichungstag der Anmeldung: 27.03.2024
(73) Patentinhaber: W.O.M. World of Medicine GmbH, 10587 Berlin (DE)
(72) Erfinder: KLUGE, Tobias, 12555 Berlin (DE); LANGEN, Fabian, 10405 Berlin (DE); HASER, Christian, 14532 Stahnsdorf (DE)
(74) Vertreter: Gulde & Partner
(86) Internationale Anmeldenummer: PCT/IB2022/054543
(87) Internationale Veröffentlichungsnummer: WO 2022/243844

(56) Entgegenhaltungen:
- US-A1- 2015 112 246
- US-A1- 2016 106 934
- US-A1- 2020 094 002
- US-A1- 2021 016 022

## Beschreibung

Die vorliegende Erfindung betrifft eine Kupplungsvorrichtung für medizintechnische Pumpen, insbesondere für Insufflationspumpen für die Endoskopie, mittels derer mehrere Schlauchverbindungen gleichzeitig geschlossen werden können.

### Anwendungsgebiet der Erfindung

Es ist bekannt, bei endoskopischen Untersuchungen und insbesondere bei therapeutischen Eingriffen, die jeweilige Körperhöhle durch Fluidzufluss auszudehnen. Im Rahmen der Laparoskopie wird in der Regel in das Abdomen ein Gas (vorzugsweise CO₂) eingebracht und ein Innendruck erzeugt, der höher ist als der Außendruck. Auf diese Weise wird das Abdomen gedehnt, sodass Platz für die Einführung der Operationsgeräte geschaffen wird. Moderne Systeme weisen auch eine Absaugvorrichtung auf, um einerseits sichtbeeinträchtigende Rauchgase schnell entfernen zu können, andererseits aber den Druck in der Körperhöhle während der Operation möglichst konstant zu halten.

Hierbei stellt sich das Problem, den Insufflator mit verschiedenen Pumpen sowie Messleitungen mit den notwendigen Schläuchen zu kuppeln. Hierbei handelt es sich aus Hygienegründen um Einwegartikel. Die Verbindung mehrerer Schläuche mit dem Insufflator dauert Zeit. Zudem kann es möglicherweise zu Verwechselungen der Schläuche kommen, wenn bauartgleiche Kupplungen verwendet werden. Die Alternative besteht in der Verwendung unterschiedlicher Kupplungssysteme, was vom Bedienpersonal als umständlich wahrgenommen wird. Verschiedene Kupplungsvorrichtungen für Insufflationspumpen sind aus den Dokumenten US 2021/016022 A1, US 2015/112246 A1, US 2020/094002 A1 und US 2016/106934 A1 bekannt.

Zur Überwindung dieses technischen Problems wird die nachfolgend beschriebene Kupplungsvorrichtung vorgeschlagen, die gleichzeitig mehrere Schlauchverbindungen herstellen kann und dabei die Dichtheit jeder Verbindung sicherstellt.

### Grundzüge der Erfindung

Die Erfindung besteht aus einer Schlauchverbindungsvorrichtung zum Verbinden von n Schläuchen, durch die ein Gas strömt mit einem Insufflator, wobei n zwei bis fünf ist, wobei insufflatorseitig ein hülsenförmig ausgeformtes Element ausgebildet ist, wobei das hülsenförmige Element im Bereich des Hülsenbodens n Insufflationskolben als Anschlusselemente aufweist,
wobei die n Insufflationskolben als Anschlusselemente mittels Federspannung aus dem Hülsenboden herausragen und durch Druck der Schläuche in Richtung des Hülsenbodens gedrückt werden können, wodurch die Oberfläche der Insufflationskolben bei bestehender Schlauchverbindung bündig mit dem Hülsenboden ist,
wobei jeweils ein elastisches Dichtungselement zwischen jedem Anschlusselement und jedem Schlauch angeordnet ist, wobei im Bereich der Hülsenöffnung ein oder mehrere Rastelemente angeordnet sind, die ein Einrasten der Schläuche gewährleisten und die Schlauchverbindung verriegeln,
wobei schlauchseitig Anschlusselemente angeordnet sind, die zu den Anschlusselementen des Hülsenbodens kompatibel sind,
wobei ein zum hülsenförmig ausgebildeten Element kompatibles Element als Schlauchset n Hohlzylinder aufweist an die die n Schläuche angeschlossen werden, so dass eine Fluidverbindung aller n Schläuche zum Insufflator gewährleistet ist,
und wobei die Schläuche orthogonal zur Längsachse der Insufflationskolben aus dem Schlauchset austreten.

Die Schlauchverbindungsvorrichtung besteht daher aus zwei Teilen:
Einem hülsenförmigen Teil, das insufflatorseitig als Aufnahme angeordnet ist und einem damit kompatiblen Element als Steckteil, welches als Schlauchset in das hülsenförmige Aufnahmeteil gesteckt wird. Das hülsenförmige Aufnahmeteil weist nach außen Anschlüsse zum Insufflator au, innerhalb der Hülse sind n Insufflationskolben angeordnet. Das Steckteil weist nach außen Anschlüsse für n Schläuche auf, innenseitig sind n Hohlzylinder kompatibel zu den Insufflationskolben des Hülsenteils angeordnet.

Durch die Kombination der elastischen Dichtungselemente mit einem zusätzlichen und abgestimmten elastischen Element (Feder) zum Ausgleich von Positions- und Toleranzabweichungen bei Verbindung von Schlauch und Insufflator, können Toleranzen ausgeglichen und gasdichte Verbindung hergestellt werden. Auf diese Art und Weise lassen sich größere Fertigungsabweichungen tolerieren und damit Kosten durch hohe Anforderungen an die Genauigkeit von Schlauchsetkomponenten reduzieren. Toleranzen, die durch den Dichtungswerkstoff selbst aufgefangen werden, können so um ein Vielfaches erhöht werden.

Die elastischen Elemente (Federn) werden derart ausgelegt, dass zunächst eine definierte Vorspannung herrscht, welche die gewünschten Dichtkräfte garantiert. Die Federelemente besitzen eine geringe Federsteifigkeit, sodass sich die Gegenkraft bei zusätzlicher Auslenkung nur minimal erhöht.

Die Kombination von elastischen Elementen kann separat für jede zu verbindende Dichtung ausgeführt werden, um weitere Toleranzen durch die Geometrie des Schlauchsets oder verschiedenartige Dichtkonturen ausgleichen zu können.

Auf diese Weise kann ein Dichten in mehreren Ebenen ermöglicht werden.

Durch die (axiale) Verschiebung des elastischen Elementes, kann die Position jeder einzelnen Dichtungsschnittstelle detektiert/überwacht/ausgewertet werden (per Mikroschalter oder mittels anderer Positionssensorik gemäß dem Stand der Technik) und genutzt werden für
1.: Erkennung des korrekten Einlegens (Erkennung von Betätigung und korrekter Endposition)
2.: Identifizierung des eingelegten Schlauchsets (sofern Schlauchset 1 bspw. andere Dichtungen betätigt als Schlauchset 2)

Eine alternative Ausführung ist, dass mehrere Dichtungen mit nur einer elastischen Komponente konnektiert werden (eine große, federnd gelagerte "Platte", die alle nötigen Anschlüsse beinhaltet).

Während der Montage der geräteseitigen Schlauchaufnahme kann ein zusätzliches Einstellen der Rastgeometrie zur Dichtung mit Einstelllehren durch entsprechend gewählte Fertigungstoleranzen entfallen. Ebenfalls ist die Toleranz der schlauchseitigen Steckverbindung größer wahlbar, was Ebenheit und Lage betrifft.

Neben dem Ausgleich von Längentoleranzen der Dichtgeometrie wird in der Schnittstelle eine Ausrichtung des Schlauchsets vor dem ersten Kontakt mit der Dichtung vorgenommen. Diese Ausrichtung erfolgt bevor das Schlauchset die Dichtung erstmalig berührt. Mit der Ausrichtung vor Kontakt mit der Dichtung sollen Lateralbewegungen von Dichtung und Dichtgeometrie reduziert und die Standzeit der Dichtung erhöht werden. Ein falsches Einlegen wird durch eine entsprechende nur auf eine Art zusammenpassende Geometrie von Schlauchset und Gerät vermieden.

Die Verbindung des Schlauches mit dem Gerät soll möglichst mit einer Hand und einem Handgriff erfolgen. Daher entfallen Möglichkeiten eines Einlegens und Entnehmens mit anschließender Verriegelung

Die Gesamteinlegekraft kann durch die Wahl der Federvorspannung und Federkennlinien unabhängig vom Dichtungsmaterial eingestellt werden und auf Kundenwünsche adaptiert werden. So sind auch gezielte Kraftprofile und haptisches Feedback durch bspw. "voreilende" Dichtungen umsetzbar.

Das Dichtungskonzept der federnden Anschlüsse kann auch für eine nicht "reinaxiale" Einlegebewegung realisiert werden. Dann muss darauf geachtet werden, dass die Relativbewegung zwischen starrer und weicher Komponente minimal/unkritisch ist, um Beschädigungen zu vermeiden.

Das elastischen Dichtungselemente können verschiedenartig lokalisiert/positioniert werden:
In der bevorzugten Ausführung ist die starre Dichtkontur im Schlauchset und weiche (Silikon-)Dichtung sowie Federelemente im Gerät positioniert.

Denkbar ist eine alternative Ausführung, wo die Positionierung der weichen Komponente (als Verschleißteil) im Schlauchset und der starren Kontur im Gerät.

Auch eine Federung mit ähnliche Kennlinie kann in einer alternativen Ausführung im Schlauchset integriert werden durch entsprechendes Spritzgussdesign oder eine separate Komponente.

Das Schlauchset weist eine geschlossene Außenkontur als Steckverbindung auf, welche die Dichtungen der zu verbindenden Fluidwege in der Stirnfläche positioniert hat. Es können mehrere Fluidwege verbunden werden, deren Anordnung frei wählbar ist - und sich mit der Anordnung der geräteseitigen Schlauchaufnahme decken. Somit können einfach Anordnungen realisiert werden sowohl entsprechend Platzierungsanforderungen aus dem Schlauchset, sowie aus den geräteseitigen Fluidwegen oder Sensoren. Eine symmetrische Anordnung ist besonders bevorzugt, da die Kräfte so ideal verteilt werden. Jede andere Anordnung ist aber möglich und beeinträchtigt die erfindungsgemäße toleranzausgleichende Dichtung nicht.

Die Steckverbindung wird durch Einschieben in die geräteseitige Aufnahme (Hülse) realisiert. Die Schläuche treten in der erfindungsgemäßen Lösung seitlich zur Achse der Verbindungswege aus dem Verbindungsstück aus, was den Vorteil hat, dass diese nicht durch andere Gegenstände verbogen und abgeknickt werden. Durch ein Abknicken würde sich der offene Querschnitt des Schlauches reduzieren, was eine Behinderung des Durchflusses bewirkt. Die Schläuche sind in dem Steckverbindungsteil intern auf Hohlzylinder aufgeschoben und ggf. verklebt und fluidisch mit den in der Stirnfläche vorhandenen Öffnungen verbunden.

Die hülsenförmige Aufnahmevorrichtung kann Bestandteil des Insufflators sein, beispielsweise in die Stirn- oder Seitenfläche des Insufflators versenkt eingearbeitet sein.

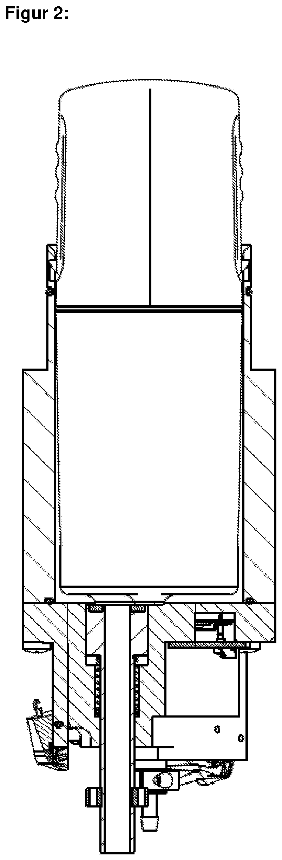

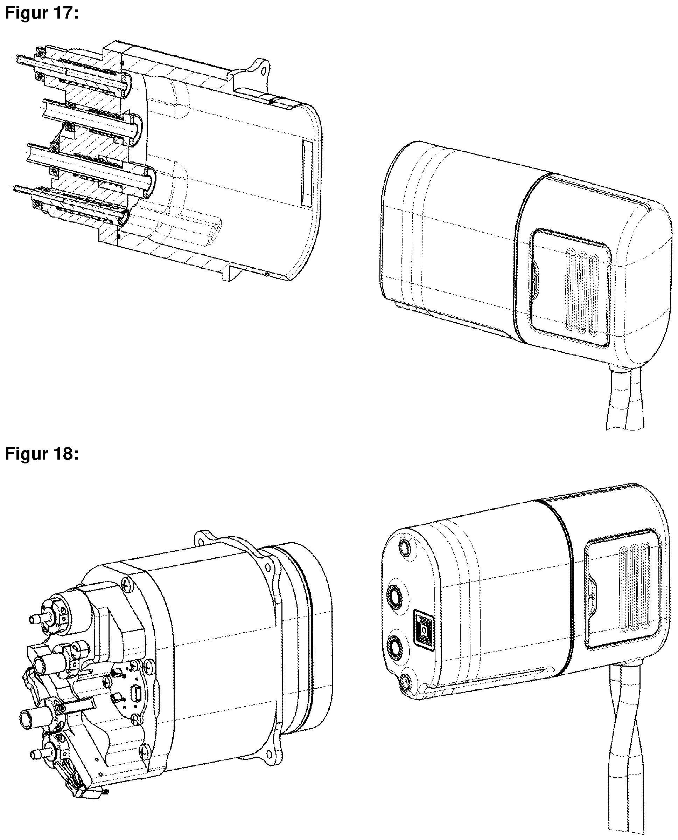

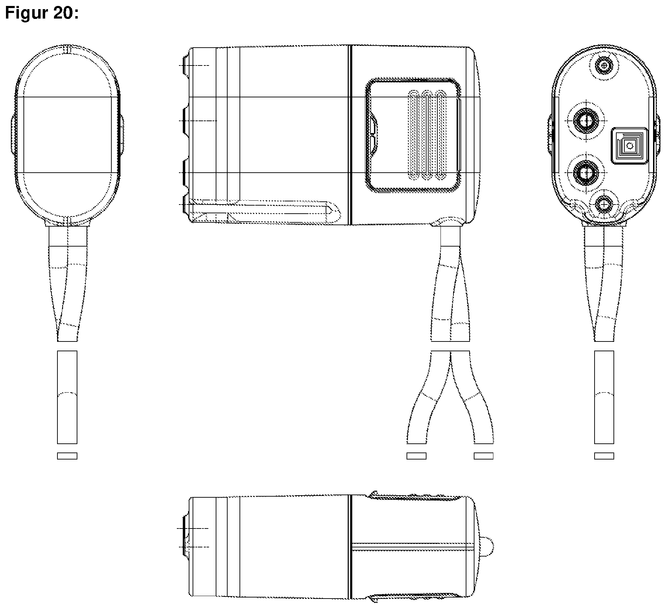

Bei der Verbindung des Steckverbindungsteils mit der geräteseitigen Aufnahme ist eine Dichtung vorauseilend, stellt also den Kontakt her und der Rest verbindet sich vor der eigentlichen Verriegelung. Dies kann genutzt werden, um ein Durchspülen der Schläuche und Ausblasen der geräteseitigen Aufnahme zu bewirken. Die Wahl welche Dichtung vorauseilend ist, ist frei und nicht durch Erfindungsmerkmale vorgegeben. Die jeweils vorgesehenen Schlauchdurchmesser bzw. Durchmesser der Dichtungen sind ebenfalls frei wählbar, jedoch haben die Durchmesser Einfluss auf die Verbindungskräfte und sind entsprechend auszulegen.

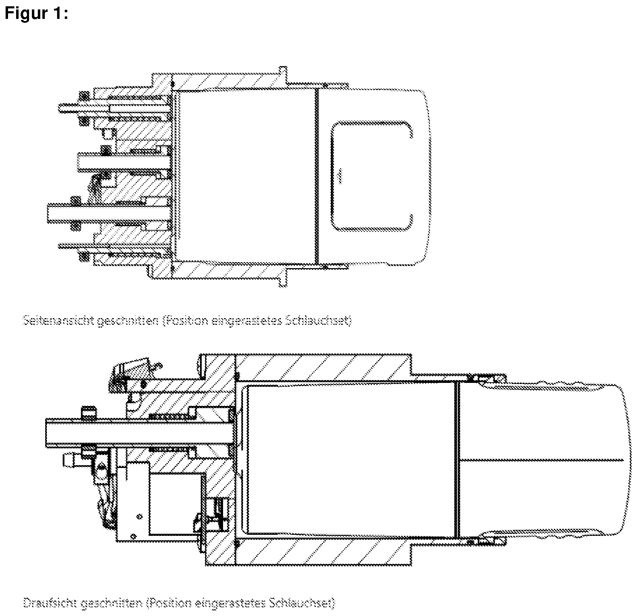

Figur 1 zeigt eine bevorzugte Ausführungsform der Erfindung mit vier Schlauchverbindungen in verschiedenen Ansichten:
   Oben: Seitenansicht mit eingerastetem Schlauchset
   Unten: Draufsicht mit eingerastetem Schlauchset
Figur 2 zeigt nochmals eine Seitenansicht. Gut zu erkennen sind drei der Schlauchanschlüsse. Ein vierter Anschluss ist in dieser Ansicht verdeckt.

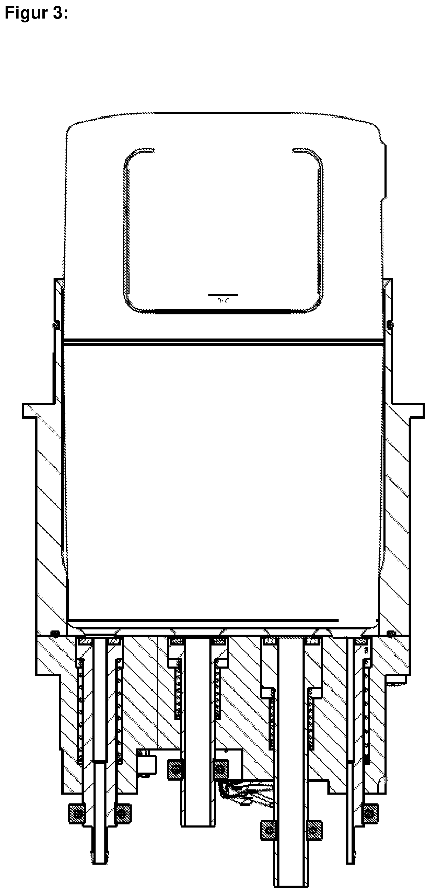

Figur 3 zeigt nochmals die Draufsicht. Gut zu erkennen sind die Rastelemente nahe der Hülsenöffnung.
Figuren 4-8 zeigen den Einlegevorgang bildlich sowie das Kraft-Weg-Verhältnis als Grafik. In den Abbildungen sind die jeweils relevanten Komponenten farbig markiert.

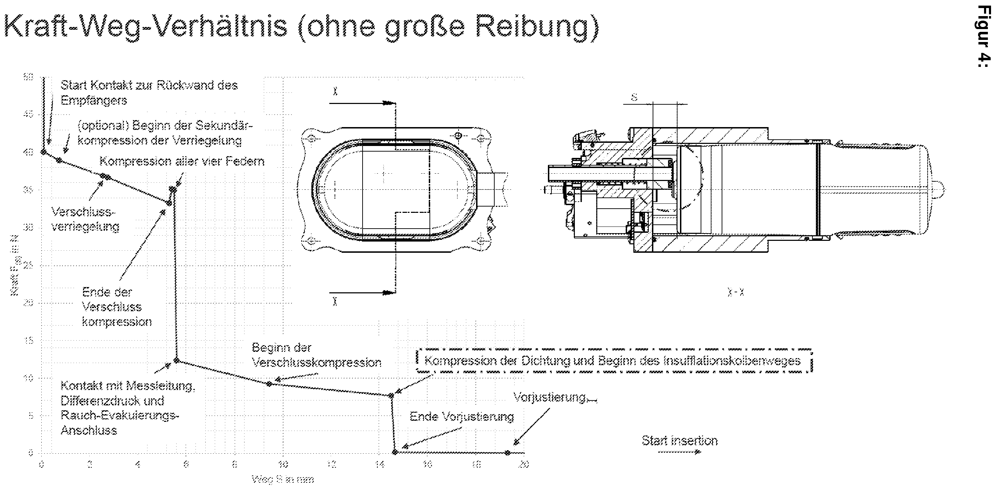

Figur 4 zeigt das Bild bei Beginn der Kompression des Dichtelementes (Kontakt von Insufflationskolben mit Schlauch)

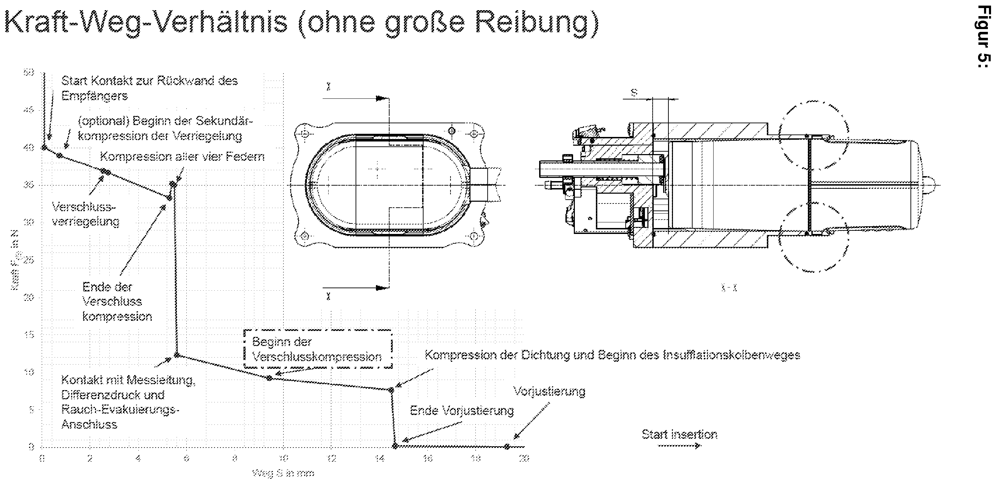

Figur 5 zeigt das Bild bei Beginn der Kompression der Rastelemente (als Verschluss der Kupplung)

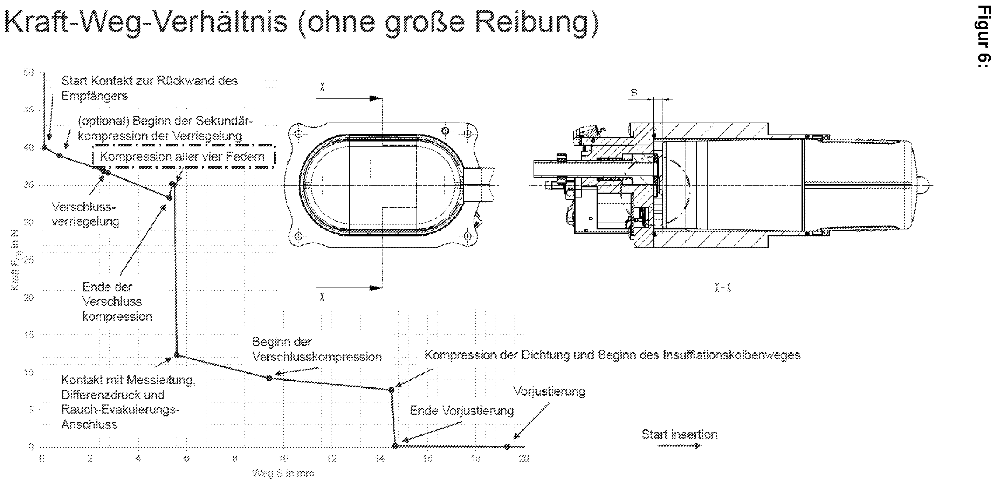

Figur 6 zeigt das Bild bei Kompression aller vier Federn

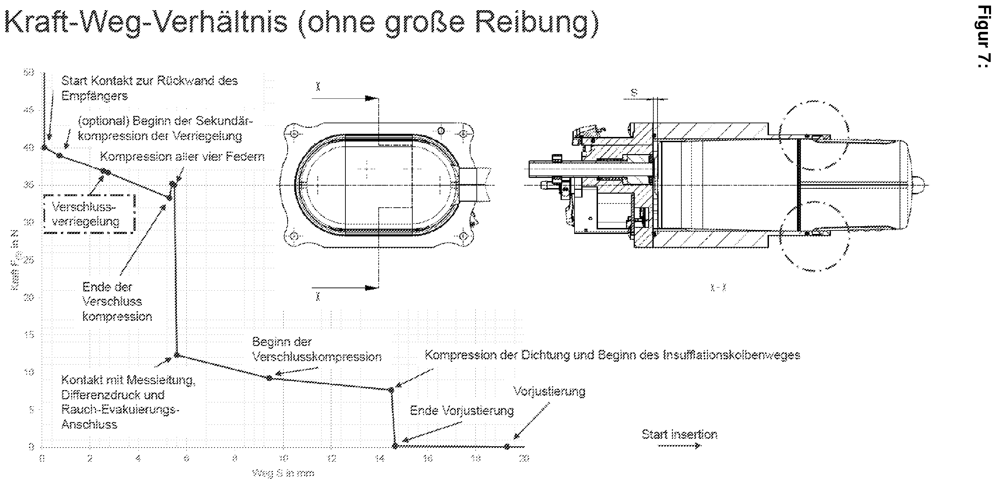

Figur 7 zeigt die Verriegelung der Rastelemente (Verschlussverriegelung)

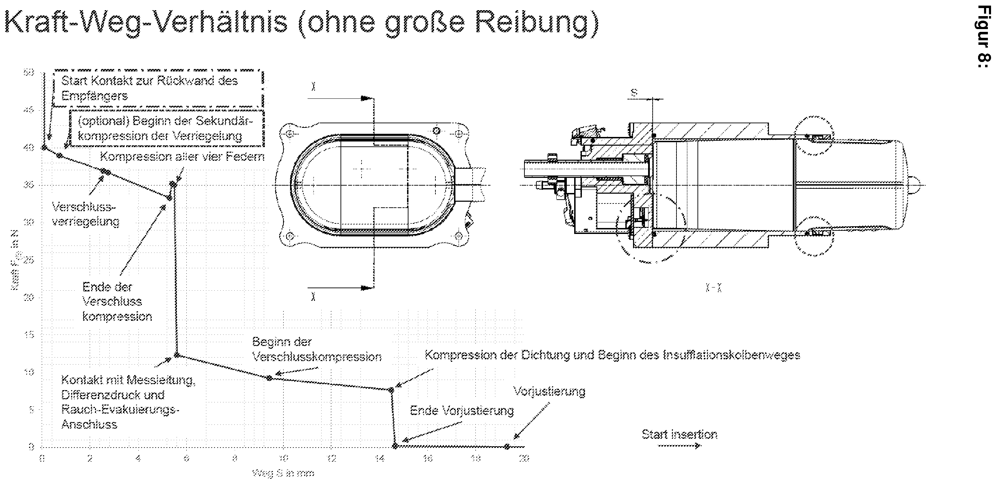

Figur 8 zeigt den Beginn der Kompression der Sekundärverriegelung die Verriegelung der Rastelemente (Verschlussverriegelung).

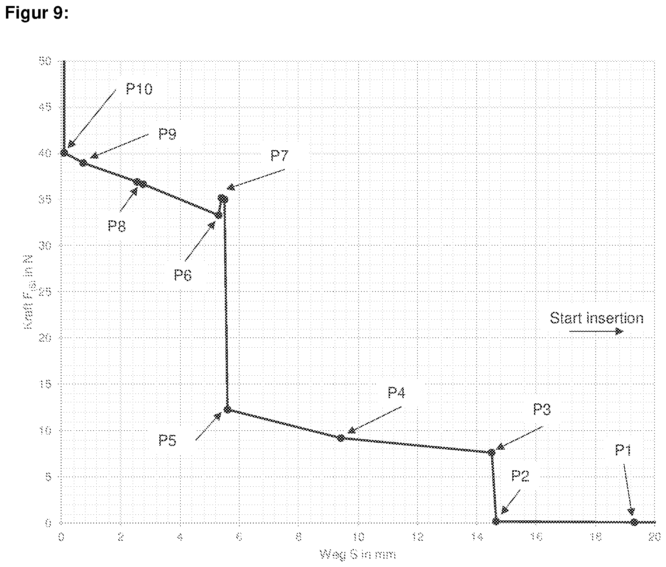

Figur 9 zeigt ein mögliches Kraftprofil. Mit den Punkten: P1 Beginn der Vorjustierung, P2 Ende der Vorjustierung, P3 Kompression der Dichtung und Beginn des Insufflationskolbenweges, P4 Beginn der Verschlusskompression, P5 Kontakt mit Messleitung, Differenzdruck und Rauch-Evakuierungs-Anschluss, P6 Ende der Verschlusskompression, P7 Kompression aller vier Federn, P8 Verschlussverriegelung, P9 (optional) Beginn der Sekundärkompression der Verriegelung, P10 Start des Kontaktes zur Rückwand des Hülsenbodens (Aufnahme, bzw. Hülse der Steckverbindung)

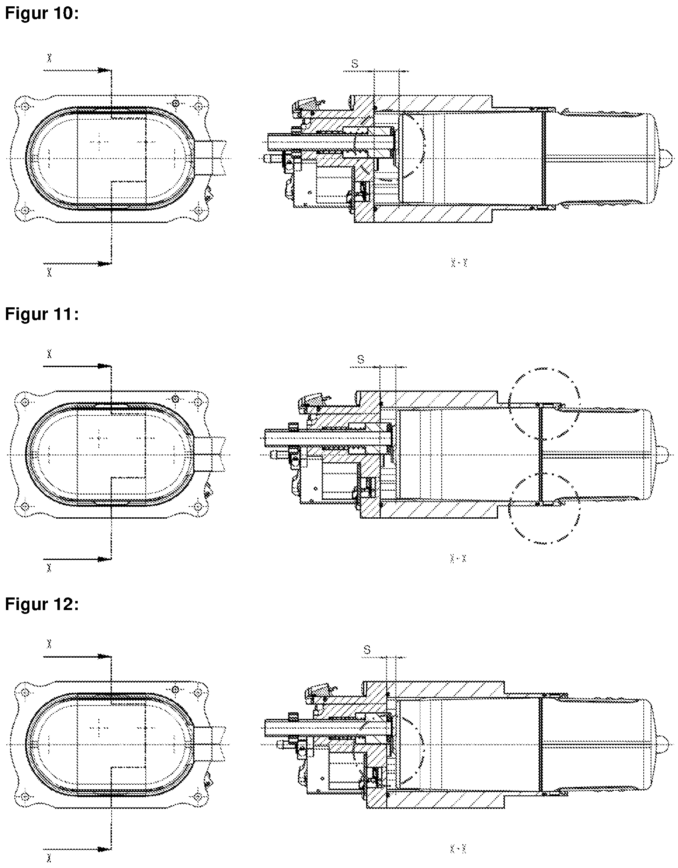

Figur 10 zeigt den Zustand des Einlegevorgangs für die Wegstrecke S am Punkt P3. Die relevanten Komponenten sind mit einer Strich-Punkt-Linie eingekreist.
Figur 11 zeigt den Zustand des Einlegevorgangs für die Wegstrecke S am Punkt P4. Die relevanten Komponenten sind mit einer Strich-Punkt-Linie eingekreist.
Figur 12 zeigt den Zustand des Einlegevorgangs für die Wegstrecke S am Punkt P7 . Die relevanten Komponenten sind mit einer Strich-Punkt-Linie eingekreist.

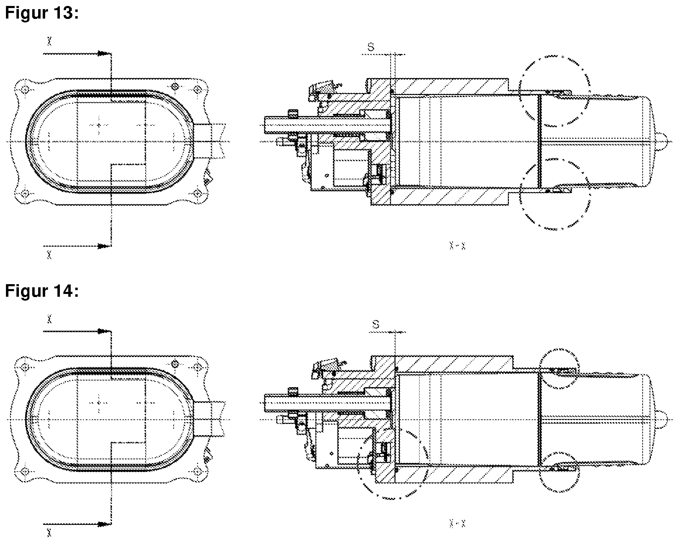

Figur 13 zeigt den Zustand des Einlegevorgangs für die Wegstrecke S am Punkt P8. Die relevanten Komponenten sind mit einer Strich-Punkt-Linie eingekreist.
Figur 14 zeigt den Zustand des Einlegevorgang für die Wegstrecke S am Punkt P9 und P10. Die relevanten Komponenten sind mit einer gestrichelten Linie für den Punkt P9 und einer Strich-Punkt-Linie für P10 eingekreist.

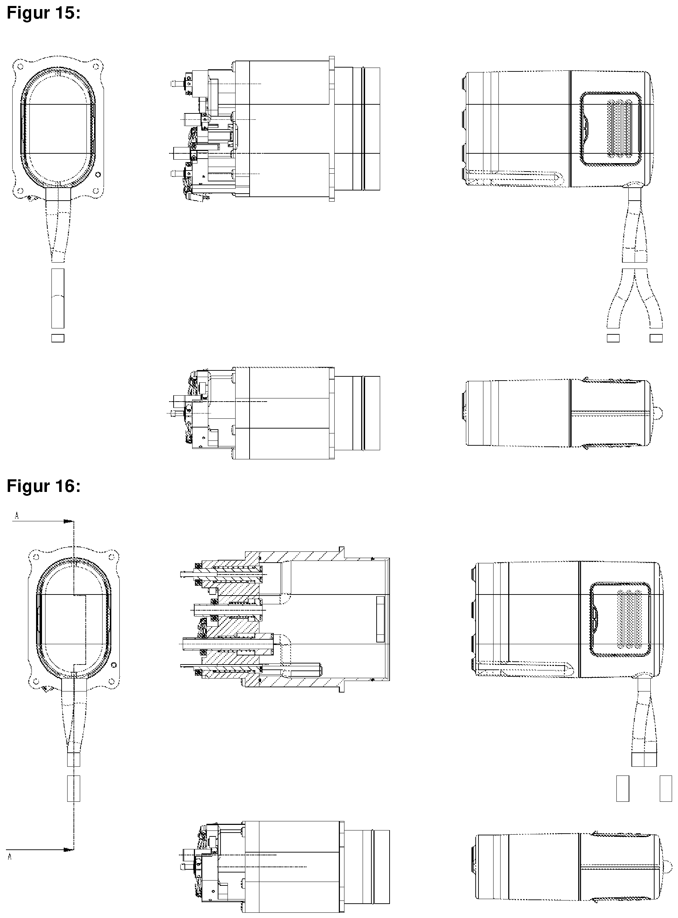

Figur 15 zeigt eine bevorzugte Ausführungsform der erfindungsgemäßen Verbindung beider Elemente (geräteseitige Hülse und Schlauchset) mit vier Schlauchverbindungen in verschiedenen Außenansichten.
   Oben links: Vorderansicht Seitenansicht mit nicht eingerastetem Schlauchset (Schlauchset verdeckt geräteseitigen Hülsenteil/Aufnahme)
   Oben rechts: Seitenansicht mit nicht eingerastetem Schlauchset
   Unten: Draufsicht mit nicht eingerastetem Schlauchset
Figur 16 zeigt eine bevorzugte Ausführungsform der erfindungsgemäßen Verbindung beider Elemente (geräteseitige Hülse und Schlauchset) mit vier Schlauchverbindungen in verschiedenen Ansichten:
   Oben links: Vorderansicht Seitenansicht mit nicht eingerastetem Schlauchset (Schlauchset verdeckt geräteseitiges Hülsenteil/Aufnahme) und Darstellung des Schnittverlaufes A-A für die geräteseitige Aufnahme
   Oben rechts: Seitenansicht mich nicht eingerastetem Schlauchset und Stufenschnitt des geräteseitigen Hülsenteils/der Aufnahme
   Unten: Draufsicht mit nicht eingerastetem Schlauchset
Figur 17 zeigt eine bevorzugte Ausführungsform der erfindungsgemäßen Verbindung beider Elemente (geräteseitige Hülse und Schlauchset) mit vier Schlauchverbindungen in räumlicher Darstellung mit nicht gestecktem Schlauchset mit Stufenschnitt des geräteseitigen Hülsenteils/der Aufnahme
Figur 18 zeigt eine bevorzugte Ausführungsform der erfindungsgemäßen Verbindung beider Elemente (geräteseitiger Hülse und Schlauchset) mit vier Schlauchverbindungen in einer weiteren räumlichen Darstellung. Gut zu erkennen sind die am Schlauchteil befindlichen und auf die Aufnahme abgestimmten Anschlüsse

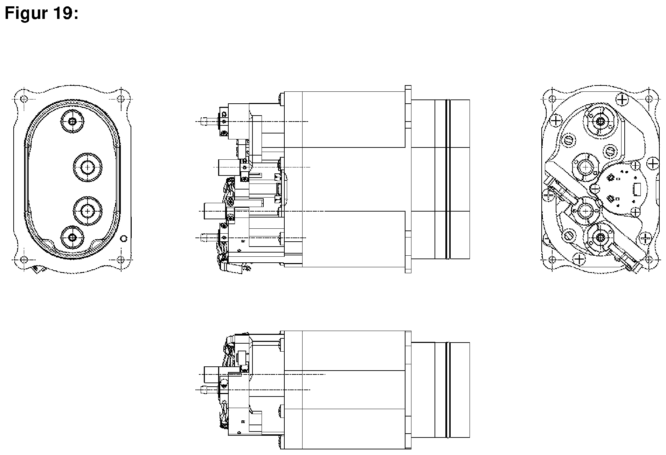

Figur 19 zeigt eine bevorzugte Ausführungsform der geräteseitigen Aufnahme des Schlauchanschlusses mit vier Anschlüssen in vier Orientierungen.
   Oben links: Vorderansicht
   Oben mitte, Seitenansicht
   Oben rechts: Rückansicht
   Unten: Draufsicht
Figur 20 zeigt eine bevorzugte Ausführungsform des Schlauchteils mit vier Anschlüssen in vier Orientierungen
   Oben links: Vorderansicht
   Oben mitte, Seitenansicht
   Oben rechts: Rückansicht
   Unten: Draufsicht

## Patentansprüche

1. Schlauchverbindungsvorrichtung zum Verbinden von n Schläuchen, durch die ein Gas strömt mit einem Insufflator, wobei n zwei bis fünf ist,
wobei insufflatorseitig ein hülsenförmig ausgeformtes Element ausgebildet ist, wobei das hülsenförmige Element im Bereich des Hülsenbodens n Insufflationskolben als Anschlusselemente aufweist,
wobei die n Insufflationskolben als Anschlusselemente mittels Federspannung aus dem Hülsenboden herausragen und durch Druck der Schläuche in Richtung des Hülsenbodens gedrückt werden können,
wobei jeweils ein elastisches Dichtungselement zwischen jedem Anschlusselement und jedem Schlauch angeordnet ist, wobei im Bereich der Hülsenöffnung ein oder mehrere Rastelemente angeordnet sind, die ein Einrasten der Schläuche gewährleisten und die Schlauchverbindung verriegeln,
wobei schlauchseitig Anschlusselemente angeordnet sind, die zu den Anschlusselementen des Hülsenbodens kompatibel sind,
wobei ein zum hülsenförmig ausgebildeten Element kompatibles Element als Schlauchset n Hohlzylinder aufweist an die die n Schläuche angeschlossen werden, so dass eine Fluidverbindung aller n Schläuche zum Insufflator gewährleistet ist,
**dadurch gekennzeichnet, dass** die Schläuche orthogonal zur Längsachse der Insufflationskolben aus dem Schlauchset austreten, und dass die Oberfläche der Insufflationskolben bei bestehender Schlauchverbindung bündig mit dem Hülsenboden ist.

2. Schlauchverbindungsvorrichtung von n Schläuchen, durch die ein Gas strömt mit einem Insufflator gemäß Anspruch 1, wobei das elastisches Dichtungselement entweder Bestandteil der Insufflationskolben als Anschlusselement oder Bestandteil der schlauchseitigen Anschlusselemente ist.

## Claims

1. A hose connecting device for connecting n hoses through which a gas flows to an insufflator, where n is two to five,
wherein a sleeve-shaped element is formed on the insufflator side,
wherein the sleeve-shaped element has n insufflation pistons as connecting elements in the region of the sleeve base,
wherein the n insufflation pistons as connecting elements protrude from the sleeve base by means of spring tension and can be pressed in the direction of the sleeve base by pressure from the hoses,
wherein an elastic sealing element is arranged between each connecting element and each hose, wherein one or more latching elements are arranged in the region of the sleeve opening, which ensure that the hoses engage and lock the hose connection,
wherein connecting elements are arranged on the hose side compatible with the connecting elements of the sleeve base,
wherein an element as a hose set compatible with the sleeve-shaped element has n hollow cylinders to which the n hoses are connected, so that a fluid connection of all n hoses to the insufflator is ensured,
**characterised in that** the hoses exit from the hose set orthogonally to the longitudinal axis of the insufflation pistons, and **in that**, in case of an existing hose connection, the surface of the insufflation pistons is flush with the sleeve base.

2. The hose connecting device for n hoses through which a gas flows with an insufflator of claim 1, wherein the elastic sealing element is either a component of the insufflation pistons as connecting element or a component of the connecting elements on the hose side.

## Revendications

1. Dispositif de raccordement de tuyaux pour raccorder n tuyaux, à travers lesquels un gaz s'écoule, à un insufflateur, n étant compris entre deux et cinq,
un élément réalisé en forme de manchon étant formé du côté insufflateur, l'élément en forme de manchon présentant n pistons d'insufflation comme éléments de raccordement dans la zone du fond de manchon,
les n pistons d'insufflation faisant saillie du fond de manchon en tant qu'éléments de raccordement au moyen d'une tension élastique et pouvant être poussés en direction du fond de manchon par la pression des tuyaux,
un élément d'étanchéité élastique étant respectivement disposé entre chaque élément de raccordement et chaque tuyau, un ou plusieurs éléments d'encliquetage étant disposés dans la zone de l'ouverture de manchon, lesquels garantissent un encliquetage des tuyaux et verrouillent le raccordement des tuyaux,
des éléments de raccordement compatibles avec les éléments de raccordement du fond de manchon étant disposés du côté tuyau,
un élément compatible avec l'élément réalisé en forme de manchon présentant, en tant qu'ensemble de tuyaux, n cylindres creux auxquels sont raccordés les n tuyaux, de sorte qu'une liaison fluidique de tous les n tuyaux vers l'insufflateur est garantie,
**caractérisé en ce que** les tuyaux sortent de l'ensemble de tuyaux orthogonalement à l'axe longitudinal des pistons d'insufflation, et **en ce que** la surface des pistons d'insufflation est à fleur du fond de manchon lorsque le raccordement de tuyaux existe.

2. Dispositif de raccordement de n tuyaux à travers lesquels un gaz s'écoule avec un insufflateur selon la revendication 1, dans lequel l'élément d'étanchéité élastique est soit un composant des pistons d'insufflation en tant qu'élément de raccordement, soit un composant des éléments de raccordement du côté tuyau.
